# EUROPÄISCHE PATENTSCHRIFT

(11) **EP 1 301 494 B1**
(45) Veröffentlichungstag und Bekanntmachung des Hinweises auf die Patenterteilung: **07.09.2005**
(21) Anmeldenummer: 01960375.2
(22) Anmeldetag: 25.06.2001
(51) Int. Cl.: C07D 273/01

(54) **VERFAHREN ZUR HERSTELLUNG VON 2-ALKYL-3-ARYL- UND -HETEROARYLOXAZIRIDINEN UND NEUE 2-ALKYL-3-ARYLOXAZIRIDINE**
METHOD FOR PRODUCING 2-ALKYL-3-ARYL- AND -HETEROARYLOXAZIRIDINES AND NOVEL 2-ALKYL-3-ARYLOXAZIRIDINES
PROCEDE DE PREPARATION DE 2-ALKYL-3-ARYL- ET -HETEROARYL OXAZIRIDINES ET NOUVELLES 2-ALKYL-3-ARYL OXAZIRIDINES

(30) Priorität: 07.07.2000 DE 10033079
(43) Veröffentlichungstag der Anmeldung: 16.04.2003
(73) Patentinhaber: LANXESS Deutschland GmbH, 51368 Leverkusen (DE)
(72) Erfinder: KLAUSENER, Alexander, 50259 Pulheim (DE); LANGER, Reinhard, 47918 Tönisvorst (DE); RATSCH, Stephan, 42651 Solingen (DE); DOCKNER, Michael, 50674 Köln (DE)
(86) Internationale Anmeldenummer: PCT/EP2001/007213
(87) Internationale Veröffentlichungsnummer: WO 2002/004432

(56) Entgegenhaltungen:
- EP-A- 0 217 269
- WO-A-00/02848
- GB-A- 743 940
- KLOC K ET AL: "Synthesis of 2-alkyl-3-aryloxaziridines" SYNTHESIS, Nr. 12, Dezember 1987 (1987-12), Seiten 1084-1087, XP002180898

## Beschreibung

Die vorliegende Erfindung betrifft ein verbessertes Verfahren zur Herstellung von 2-Alkyl-3-aryloxaziridinen, 2-C₃-C₈-Cycloalkyl-3-aryloxaziridinen oder den entsprechenden -3-heteroaryloxaziridinen durch Oxidation der entsprechenden Aryl- oder Heteroarylaldimine mit einer Perverbindung.

Beispielsweise 2-tert.-Butyl-3-phenyloxaziridin dient als Ausgangsverbindung für die Herstellung von N-(tert.-Butyl)-hydroxylamin, das für die Synthese von Pharmawirkstoffen gebraucht wird.

Es ist bereits bekannt (J. Am. Chem. Soc. 79, 5739 (1957)), dass man Arylaldimine, die eine unsubstituierte oder eine mit einer Nitrogruppe substituierte Phenylgruppe enthalten, mit wasserfreier Peressigsäure in das entsprechende Oxaziridin überführen, dieses durch Destillation isolieren, anschließend mit wässrig-methanolischer Schwefelsäure hydrolysieren und durch Zugabe von Alkali das entsprechende freie, N-alkylierte Hydroxylamin erhalten kann. Die benötigte wasserfreie Peressigsäure wird in situ aus 90 %igen wässrigem Wasserstoffperoxid mit überschüssigem Essigsäureanhydrid und katalytischen Mengen Schwefelsäure in Methylenchlorid als Lösungsmittel hergestellt. Die Handhabung derartig konzentrierter WasserstoffperoxidLösungen ist sicherheitstechnisch mit außerordentlich hohem Aufwand verbunden. Man versucht deshalb die Herstellung und Handhabung großer Mengen solcher Wasserstoffperoxid-Lösung wenn irgend möglich zu vermeiden.

Aus der EP-B 0 217 269 ist bekannt, dass man zu N-Alkyl-substituierten Aryloxaziriden durch Umsetzung von Arylaldiminen mit Perpropionsäure in Benzol gelangen kann. Um die Hydrolyse der Arylaldimine und der Aryloxaziridine unter den sauren Reaktionsbedingungen auszuschließen, muss der Wassergehalt der Perpropionsäure weniger als 0,1 Gew.-% betragen. (sog. wasserfreie Perpropionsäure). Die Herstellung und Handhabung von wasserfreier Perpropionsäure ist ebenfalls ein Verfahren, das hohen sicherheitstechnischen Aufwand erfordert, insbesondere bei der azeotropen Destillation zur Entfernung des Wassers.

Die kommerziell erhältliche m-Chlorperbenzoesäure ist ein Feststoff und deshalb im Vergleich zur Peressigsäure und zur Perpropionsäure leichter handhabbar. Auch sie ist zur Oxidation von Arylaldiminen verwendet worden (J.Chem. Soc. Perkin Trans 1 1990, 301 und 2391), wobei die Umsetzung in Methylenchlorid oder Methanol, also nicht in einem wässrigen Medium erfolgt.

Bekannt ist auch die Oxidation von Benzylidenaminosäureestem zu den entsprechenden Oxaziridinen mit Monoperphthalsäure in Diethylether als Lösungsmittel (Tetrahedron Lett. 28, 2453 (1994)).

Generell gestaltet sich die Herstellung und Handhabung von Percarbonsäuren sicherer, wenn Wasser bei ihrer Herstellung und ihrer Anwendung, z.B. bei Oxidationen, zugegen ist. Jedoch neigen Imine in Gegenwart von Säuren und Wasser im Allgemeinen zur Hydrolyse. Dies erklärt, weshalb die Gegenwart von Wasser bei den bekannten Iminoxidationen weitestgehend ausgeschlossen wurde.

Die Präsenz von Säure und Wasser im Reaktionsmedium zur Herstellung von Oxaziridinen fördert auch die hydrolytische Spaltung der gebildeten Oxaziridine in den entsprechenden Aldehyd und das entsprechende N-substituierte Hydroxylamin. Letzteres kann wiederum sehr leicht durch vorhandene Percarbonsäure zu entsprechenden Nitrosoverbindung oxidiert werden. Nitrosoverbindungen sind als stark cancerogene Stoffe bekannt. Sie müssen deshalb bei der Herstellung von Zwischenprodukten für Pharmawirkstoffe ausgeschlossen sein.

Gemäß der WO 00/02848 wird das Problem der Hydrolyse und die Bildung von unerwünschten Folgeprodukten dadurch gelöst, dass man die Oxidation mit m-Chlorperbenzoesäure in einem zweiphasigen System aus Toluol und einer wässrigen Natriumcarbonatlösung durchführt. Doch ist für die Realisierung eines technischen Verfahrens eine solche Vorgehensweise nicht empfehlenswert, da zweiphasige Reaktionssysteme zu Upscaling-Problemen führen, die, wenn überhaupt, nur mit besonderem Aufwand zu lösen sind.

Alle bekannten Methoden zur Herstellung von Aryloxaziridinen durch Oxidation von Arylaminen mit Percarbonsäuren sind für eine Durchführung in technischem Maßstab nicht befriedigend. Es besteht daher immer noch ein Bedürfnis nach einem einfachen, wirtschaftlichen und risikoarmen Verfahren zur Herstellung von 2-Alkyl-3-aryl- und -heteroaryloxaziridinen.

Es wurde nun ein Verfahren zur Herstellung von 2-Alkyl-3-aryloxaziridinen, 2-C₃-C₈-Cycloalkyl-3-aryloxaziridinen und den entsprechenden -3-heteroaryloxaziridinen gefunden, das dadurch gekennzeichnet ist, dass man entsprechende N-Alkylaryl- oder -heteroarylaldimine mit einer aromatischen Percarbonsäure oder deren Salz in Gegenwart einer wasserlöslichen Base bei Temperaturen unter 30°C oxidiert, wobei als Lösungsmittel ausschließlich Wasser und ein mit Wasser mischbares Lösungsmittel eingesetzt werden und wobei alle genannten Alkyl-, Aryl- oder Heteroaryl-Reste gegebenenfalls ein- oder mehrfach substituiert sein können.

Als N-Alkyl-arylaldimine, N-C₃-C₈-Cycloalkyl-arylaldimine und entsprechende -heteroarylaldimine kommen z.B. solche der Formel (I) in Frage 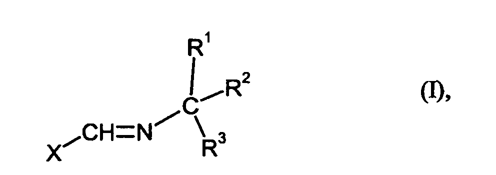 in der
- R¹, R² und R³: unabhängig voneinander für Wasserstoff, geradkettiges oder verzweigtes C₁-C₂₀-Alkyl, C₃-C₈-Cycloalkyl, geradkettiges oder verzweigtes C₂-C₁₀-Alkenyl oder C₆-C₁₀-Aryl stehen, oder
die gesamte C(R¹)(R²)(R³)-Gruppe für einen C₃-C₈-Cycloalkylrest steht und
- X: für C₆-C₁₂-Aryl oder Heteroaryl mit 4 bis 5 C-Atomen und 1 bis 2, gleichen oder verschiedenen Heteroatomen aus der Reihe N, O und S steht,
wobei alle genannten Alkyl-, Cycloalkyl-, Alkenyl-, Aryl- und Heteroaryl-Reste gegebenenfalls ein- oder mehrfach substituiert sein können,
Alkylreste z.B. durch gesättigtes C₃-C₁₂-Cycloalkyl, C₆-C₁₀-Aryl, C₂-C₈-Alkinyl, Fluor, Chlor, Brom, Iod, Hydroxy, C₁-C₆-Alkoxy, C₆-C₁₀-Aryloxy, Carboxy, C₁-C₆-Alkoxycarbonyl, Nitro, Amido, Nitril, Sulfonyl oder Phosphat und
Cycloalkyl-, Alkenyl-, Aryl- und Heteroaryl-Reste z.B. durch C₁-C₆-Alkyl, Fluor, Chlor, Brom, Hydroxy, C₁-C₆-Alkoxy, Carboxy, C₁-C₆-Alkoxycarbonyl, Nitro, Sulfonyl oder Nitril.

Bei Alkenylresten handelt es sich vorzugsweise um solche mit elektronenarmen Doppelbindungen, bei Heteroarylresten vorzugsweise um solche, die als Heteroatom(e) nur Sauerstoff enthalten.

Wenn man N-Alkyl-arylaldimine, N-C₃-C₈-Cycloalkylarylaldimine oder die entsprechenden -heteroarylaldimine der Formel (I) einsetzt, erhält man die entsprechenden 2-Alkyl-3-aryloxaziridine, 2-C₃-C₈-Cycloalkyl-3-aryloxaziridine oder die entsprechenden -3-heteroaryloxaziridine der Formel (II) 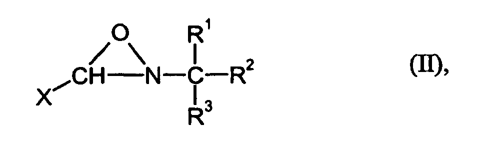 in der die verwendeten Symbole die bei Formel (I) angegebene Bedeutung haben.

In den Formeln (I) und (II) stehen R¹, R² und R³ bevorzugt unabhängig voneinander für Wasserstoff, geradkettiges oder verzweigtes C₁-C₁₀-Alkyl, C₃-C₆-Cycloalkyl, geradkettiges oder verzweigtes C₂-C₆-Alkenyl oder Phenyl oder die gesamte C(R¹)(R²)(R³)-Gruppe für C₃-C₆-Cycloalkyl, wobei die genannten Reste nicht weiter substituiert sind.

In den Formeln (I) und (II) steht X bevorzugt für Phenyl, Naphthyl oder Furyl, wobei Phenyl- und Naphthyl-Reste gegebenenfalls durch einen oder zwei gleiche oder verschiedene Reste aus der Gruppe C₁-C₆-Alkyl, Fluor, Chlor, Brom, Hydroxy, C₁-C₆-Alkoxy, C₁-C₆-Alkoxycarbonyl, Nitro, Sulfonyl und Nitril substituiert sein können.

In den Formeln (I) und (II) stehen die gesamte C(R¹)(R²)(R³)-Gruppe besonders bevorzugt für unsubstituiertes n-Propyl, n-Butyl, iso-Butyl, t-Butyl, n-Pentyl, Cyclopropyl, Cyclopentyl oder Cyclohexyl und X besonders bevorzugt für 4-Methoxyphenyl, 4-Methylphenyl, 4-Chlorphenyl oder 4-Nitrophenyl.

Als aromatische Percarbonsäuren und deren Salze kommen insbesondere m-Chlorperbenzoesäure, Monoperoxyphthalsäure und deren Alkali- und Magnesiumsalze in Frage. Bevorzugt verwendet man das Magnesiumsalz der Monoperoxyphthalsäure.

Als wasserlösliche Basen kommen beispielsweise in Frage: Alkali- und Erdalkalioxide, -hydroxide, -carbonate, -bicarbonate, -hydrogenphosphate und -dihydrogenphosphate. Bevorzugt verwendet man Natriumcarbonat oder Natriumbicarbonat.

Als mit Wasser mischbare Lösungsmittel kommen z.B. unter den Reaktionsbedingungen inerte, mit Wasser mischbare organische Lösungsmittel in Frage, beispielsweise ein- und mehrwertige Alkohole mit bis zu 6 C-Atomen. Bevorzugt verwendet man Methanol oder Ethanol.

Man kann das erfindungsgemäße Verfahren so durchführen, dass man zunächst eine Mischung aus dem N-Alkyl-arylaldimin, N-C₃-C₈-Cycloalkyl-arylaldimin- oder einem entsprechenden -heteroarylaldimin und dem mit Wasser mischbaren Lösungsmittel herstellt. Der Gehalt an dem Aldimin in dem Lösungsmittel kann in sehr weiten Bereichen variiert werden. Die Konzentration des Aldimins im Gemisch mit dem Lösungsmittel, kann z.B. 5 bis 80 Gew.-% betragen, bevorzugt liegt sie bei 20 bis 50 Gew.-%, insbesondere bei 35 bis 40 Gew.-%.

Diese Mischung kann man dann mit einer wässrigen Lösung der Base bei beispielsweise 0 bis 30°C versetzen. Bevorzugt macht man dies bei 10 bis 25°C. Die Konzentration der Base im Wasser ist beliebig variierbar und kann beispielsweise 15 bis 30 Gew.-% betragen. Bevorzugt sind 15 bis 25 gew.-%ige Lösungen. Sekundäre und tertiäre Amine kann man auch als solche, d.h. ohne Wasser, einsetzen. Die Menge der Base ist jedoch vorzugsweise in engen Grenzen zu halten. Beispielsweise kann man bezogen auf 1 Mol des Aldimins 0,09 bis 2 Äquivalente Base einsetzen. Bevorzugt werden 0,9 bis 1,2 Äquivalente Base, insbesondere 1,0 bis 1,1 Äquivalente Base eingesetzt.

Zu der so erhaltenen Mischung kann man eine Lösung der aromatischen Percarbonsäure oder deren Salz in Wasser zufügen. Die Dosiergeschwindigkeit wird so gewählt, dass trotz der abzuführenden Reaktionswärme die Reaktionstemperatur 30°C nicht übersteigt. Die Untergrenze der Reaktionstemperatur kann beispielsweise bei 0°C liegen. Bevorzugt sind Reaktionstemperaturen im Bereich 10 bis 25°C. Bezogen auf 1 Mol des Aldimins kann man z.B. 0,9 bis 1,2 Äquivalente aktiven Sauerstoff in Form der aromatischen Percarbonsäure oder deren Salzen einsetzen. Bevorzugt liegt diese Menge bei 0,95 bis 1,05 Äquivalenten. Häufig ist im Handel als aromatische Percarbonsäure oder deren Salz kein 100 %iges Material erhältlich. Auch nicht 100 %ige aromatische Percarbonsäuren und deren Salze sind für das erfindungsgemäße Verfahren geeignet. Vorzugsweise werden aromatische Percarbonsäure und deren Salze eingesetzt, die einen Gehalt an mindestens 80 % aufweisen.

Die Konzentration der Lösung der aromatischen Carbonsäure oder deren Salze in Wasser kann z.B. zwischen 1 und 20 Gew.-% betragen. Vorzugsweise liegt sie bei 15 bis 20 Gew.-%.

Nach beendeter Zugabe der wässrigen Lösung einer aromatischen Percarbonsäure oder deren Salze kann man die Reaktionsmischung bei z.B. 5 bis 30°C, bevorzugt bei 15 bis 25°C rühren, bis das Reaktionsende z.B. mittels Gaschromatographie festgestellt worden ist. Das Reaktionsende gilt als erreicht, wenn man gaschromatographisch nur noch maximal 0,5 Flächenprozent des Eduktes feststellt. Im Allgemeinen ist das nach 2 bis 3 Stunden der Fall.

Zur Aufarbeitung des Reaktionsgemisches genügt es häufig, den Rührer abzustellen, worauf sich das Gemisch in zwei Phasen trennt, eine obere Produktphase und eine untere wässrige Phase. Durch Phasentrennung kann man dann das hergestellte 2-Alkyl-3-aryloxaziridin, 2-C₃-C₈-Cycloalkyl-3-aryloxaziridin - oder das entsprechende -3-heteroaryloxaziridin erhalten.

In manchen Fällen ist die Phasentrennung des Reaktionsgemisches erschwert, z.B. wenn Mischphasen auftreten oder die eingesetzte aromatische Percarbonsäure oder deren Salz Zusätze enthalten hat (etwa Stabilisatoren), die einer Phasentrennung entgegenwirken. Im Falle von Mulm-Phasen kann häufig durch Filtration ein Filtrat erhalten werden, bei dem die Phasentrennung besser erfolgt. In anderen Fällen sollte man versuchen, aromatische Percarbonsäuren oder deren Salze zu verwenden, die keine bei der Phasentrennung störenden Zusätze enthalten oder eine Extraktionsmethode zur Aufarbeitung des Reaktionsgemisches anwenden.

Es ist auch möglich, das Produkt extraktiv mit Hilfe eines organischen Lösungsmittels aus dem Reaktionsgemisch zu gewinnen und anschließend aus dem Extrakt das Lösungsmittel destillativ abzutrennen. Zur Extraktion können z.B. gesättigte und ungesättigte Kohlenwasserstoffe und halogenierte, gesättigte und ungesättigte Kohlenwasserstoffe verwendet werden. Bevorzugt sind Petrolether, Toluol, Methylenchlorid und Chlorbenzol.

Das in einer Ausbeute von ca. 80 % der Theorie (berechnet auf 100 %iges 2-Alkyl-3-aryloxaziridin, 2-C₃-C₈-Cycloalkyl-3-aryloxaziridin - oder entsprechendes -3-heteroaryloxaziridin isolierbare Produkt kann als Verunreinigungen neben Benzaldehyd noch Spuren des Edukts und des eingesetzten, mit Wasser mischbaren Lösungsmittels enthalten.

Das erfindungsgemäße Verfahren ist auf einfache Weise durchführbar, bei Einhaltung der angegebenen Reaktionsbedingungen ergeben sich keine besonderen sicherheitstechnischen Probleme, die erzielbaren Ausbeuten sind gut, unerwünschte Begleitstoffe entstehen nicht und Upscaling-Probleme sind ohne besonderen Aufwand zu lösen. Es konnte nicht erwartet werden, dass das erfindungsgemäße Verfahren so gute Ergebnisse liefert, denn wie eingangs aufgezeigt, war zu befürchten, das unter der Reaktionsbedingungen mit der Spaltung des gebildeten Oxaziridins und der Bildung unerwünschter Nebenprodukte zu rechnen ist.

Durch das erfindungsgemäße Verfahren gelingt es, die neuen Verbindungen 2-Isopropyl-3-(4-methoxyphenyl)-oxaziridin und 2-n-Propyl-3-(4-methoxyphenyl)-oxaziridin herzustellen. Sie eröffnen den Zugang zu den entsprechenden Hydroxylaminen und damit zu neuen Synthesebausteinen für Pharmawirkstoffe.

### Beispiele

### Beispiel 1

In einem 2 l Doppelmantelplanschliffbecherglas mit Bodenablass, Glasrührer, Temperaturfühler, Tropftrichter und Kühler wurden 140,6 g N-(tert.-Butyl)-benzaldimin und 222,3 g Methanol bei Raumtemperatur vorgelegt. Zu dieser Lösung tropfte man innerhalb von 30 Minuten 523,4 g einer 17 gew.-%igen wässrigen Natriumcarbonatlösung bei 18 bis 22°C Innentemperatur zu. Nach beendeter Zugabe wurde die Manteltemperatur des Reaktors mit Hilfe eines Thermostaten auf 15°C eingestellt. Sobald diese Temperatur erreicht war, wurden innerhalb einer Stunde 1323,1 g Magnesiummonoperoxyphthalat-hexahydrat in Form einer frisch hergestellten 20 gew.-%igen wässrigen Lösung, aufgeteilt in zwei gleich große Portionen, zudosiert. Dabei wurde kontrolliert, dass eine Innentemperatur von 23°C nicht überschritten wurde. Die sich bildende Emulsion wurde bei einer Innentemperatur von 22 bis 23°C und einer Manteltemperatur von 20°C 3 Stunden gerührt. Danach wurde der Rührer abgestellt und nach einer Stunde und zwanzig Minuten die obere Produktphase von der unteren wässrigen Phase abgetrennt. So wurden 129,6 g einer gelben Flüssigkeit erhalten. Das entspricht einer Rohausbeute von 85,6 % der Theorie an 2-tert.-Butyl-3-phenyl-oxaziridin. Die gaschromatographische Analayse des isolierten Produktes ergab:

| | |
|---|---|
| 2-tert.-Butyl-3-phenyloxaziridin | 95,9 Flächen-% |
| N-(tert.-Butyl)-benzaldimin | 0,2 Flächen-% |
| Benzaldehyd | 3,5 Flächen-%. |

Der Gehalt an 2-tert.-Butyl-3-phenyloxaziridin im isolierten Produkte betrug gemäß ¹H-NMR-Analyse 92,2 Gew.-%.

### Beispiel 2

In einem Mehrhalskolben mit Rührer, Thermometer, Tropftrichter und Kühler wurden 17,7 g N-(Isopropyl)-4-methoxybenzaldimin und 25,7 g Methanol bei Raumtemperatur vorgelegt. Zu der Lösung tropfte man innerhalb von 35 Minuten 62,4 g einer 17 gew.%-igen wässrigen Natriumcarbonatlösung bei 18-22°C Innentemperatur zu. Innerhalb einer Stunde wurden 155,0 g Magnesiummonoperoxyphtalat-hexahydrat in Form einer 20 gew.%-igen wässrigen Lösung, aufgeteilt in zwei gleich große Portionen, zudosiert. Mittels eines Eis-/Wasserbades wurde kontrolliert, dass eine Innentemperatur von 23 °C nicht überschritten wurde. Es wurde 3 Stunden bei 22 bis 23°C gerührt. Nach Extraktion der Reaktionsmischung mit 200 ml Methylenchlorid und Entfernen des Lösungsmittels im Vakuum wurden 17,8 g 2-Isopropyl-3-(4-methoxyphenyl)-oxaziridin erhalten. Das entspricht einer Ausbeute von 92 %.
¹H-NMR (400 MHz, CDCl₃): δ 1.15 (3 H, d, J 6 Hz, CH₃-iPr), 1.31 (3 H, d, J 6 Hz, CH₃-iPr), 2.25-2.39 (1 H, m, CH-iPr), 3.79 (3 H, s, CH₃O), 4.45 (0.9 H, s, E-Isomer), 5.22 (0.1 H, s, Z-Isomer), 6.89 (2 H, d, J 10 Hz, Ar-H), 7.35 (2 H, d, J 10 Hz, Ar-H).

### Beispiel 3

In einem Mehrhalskolben mit Rührer, Thermometer, Tropftrichter und Kühler wurden 46,6 g N-(Propyl)-4-methoxybenzaldimin und 64,3 g Methanol bei Raumtemperatur vorgelegt. Zu der Lösung tropfte man 156,3 g einer 17 gew.%-igen wässrigen Natriumcarbonatlösung bei 18-22°C Innentemperatur zu. Innerhalb einer Stunde wurden 387,2 g Magnesiummonoperoxyphtalat-hexahydrat in Form einer 20 gew.%-igen wässrigen Lösung, aufgeteilt in zwei gleich große Portionen, zudosiert. Mittels eines Eis-/Wasserbades wurde kontrolliert, dass eine Innentemperatur von 23°C nicht überschritten wurde. Es wurde 3 Stunden bei 22 bis 23°C gerührt. Nach Extraktion der Reaktionsmischung mit 400 ml Methylenchlorid wurde die organische Phase mit Magnesiumsulfat getrocknet und das Lösungsmittel im Vakuum entfernt.

Es wurden 46,9 g 2-n-Propyl-3-(4-methoxyphenyl)-oxaziridin erhalten. Das entspricht einer Ausbeute von 97 %.
¹H-NMR (400 MHz, CDCl₃): δ 1.00 (3 H, t, J 9 Hz, CH₃-Pr), 1.67-1.83 (2 H, m, CH₂-Pr), 2.64-2.77 (1 H, m, CH₂-Pr), 2.83-2.97 (1 H, m, CH₂-Pr), 3.80 (3 H, s, CH₃O), 4.44 (1 H, s, E-Isomer), 5.20 (0.1 H, s, Z-Isomer), 6.89 (2 H, d, J 10 Hz, Ar-H), 7.33 (2 H, d, J 10 Hz, Ar-H).

### Beispiel 4

In einem Mehrhalskolben mit Rührer, Thermometer, Tropftrichter und Kühler wurden 19,2 g N-(Propyl)-4-nitrobenzaldimin und 90 g Methanol bei Raumtemperatur vorgelegt. Zu der Lösung tropfte man 62,4 g einer 17 gew.%-igen wässrigen Natriumcarbonatlösung bei 18-22°C Innentemperatur zu. Innerhalb einer Stunde wurden 155,0 g Magnesiummonoperoxyphtalat-hexahydrat in Form einer 20 gew.%-igen wässrigen Lösung, aufgeteilt in zwei gleich große Portionen, zudosiert. Mittels eines Eis-/Wasserbades wurde kontrolliert, dass eine Innentemperatur von 23°C nicht überschritten wurde. Es wurde 5 Stunden bei 22 bis 23°C gerührt. Nach Extraktion der Reaktionsmischung mit 250 ml Methylenchlorid wurde die organische Phase zweimal mit jeweils 100 ml Wasser gewaschen, anschließend mit Magnesiumsulfat getrocknet und das Lösungsmittel im Vakuum entfernt. Es wurden 20,4 g 2-n-Propyl-3-(4-nitrophenyl)-oxaziridin erhalten. Das entspricht einer Ausbeute von 98 %.
¹H-NMR (400 MHz, CDCl₃): δ 1.03 (3 H, t, J 9 Hz, CH₃-Pr), 1.70-1.86 (2 H, m, CH₂-Pr), 2.67-2.81 (1 H, m, CH₂-Pr), 2.92-3.10 (1 H, m, CH₂-Pr), 4.60 (0.9 H, s, E-Isomer), 5.32 (0.1 H, s, Z-Isomer), 7.60 (2 H, d, J 10 Hz, Ar-H), 8.22 (2 H, d, J 10 Hz, Ar-H).

## Patentansprüche

1. Verfahren zur Herstellung von 2-Alkyl-3-aryloxaziridinen, 2-C₃-C₈-Cycloalkyl-3-aryloxaziridinen oder den entsprechenden -3-heteroaryloxaziridinen, **dadurch gekennzeichnet, dass** man entsprechende N-Alkyl-arylaldimine, N-C₃-C₈-Cycloalkyl-arylaldimine oder entsprechende - heteroarylaldimine mit einer aromatischen Percarbonsäure oder deren Salz in Gegenwart einer wasserlöslichen Base bei Temperaturen unter 30°C oxidiert, wobei als Lösungsmittel ausschließlich Wasser und ein mit Wasser mischbares Lösungsmittel eingesetzt werden und wobei alle genannten Alkyl-, Aryl- oder Heteroaryl-Reste gegebenenfalls ein- oder mehrfach substituiert sein können.

2. Verfahren nach Anspruch 1, **dadurch gekennzeichnet, dass** man ein N-Alkylarylaldimin, N-C₃-C₈-Cycloalkyl-arylaldimin oder entsprechendes - heteroarylaldimin der Formel (I) einsetzt 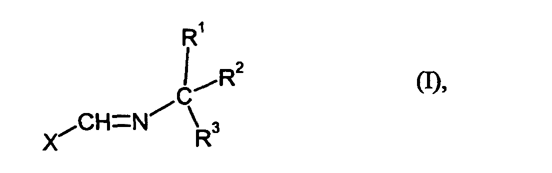 in der
R¹, R² und R³ unabhängig voneinander für Wasserstoff, geradkettiges oder verzweigtes C₁-C₂₀-Alkyl, C₃-C₈-Cycloalkyl, geradkettiges oder verzweigtes C₂-C₁₀-Alkenyl oder C₆-C₁₀-Aryl stehen, oder
die gesamte C(R¹)(R²)(R³)-Gruppe für einen C₃-C₈-Cycloalkylrest steht und
X für C₆-C₁₂-Aryl oder Heteroaryl mit 4 bis 5 C-Atomen und 1 bis 2, gleichen oder verschiedenen Heteroatomen aus der Reihe N, O und S steht,
wobei alle genannten Alkyl-, Cycloalkyl-, Alkenyl-, Aryl- und Heteroaryl-Reste gegebenenfalls ein- oder mehrfach substituiert sein können,
Alkylreste durch gesättigtes C₃-C₁₂-Cycloalkyl, C₆-C₁₀-Aryl, C₂-C₈-Alkinyl, Fluor, Chlor, Brom, Iod, Hydroxy, C₁-C₆-Alkoxy, C₆-C₁₀-Aryloxy, Carboxy, C₁-C₆-Alkoxycarbonyl, Nitro, Amido, Nitril, Sulfonyl oder Phosphat und
Cycloalkyl-, Alkenyl-, Aryl- und Heteroaryl-Reste durch C₁-C₆-Alkyl, Fluor, Chlor, Brom, Hydroxy, C₁-C₆-Alkoxy, Carboxy, C₁-C₆-Alkoxycarbonyl, Nitro, Sulfonyl oder Nitril
und die entsprechenden 2-Alkyl-3-aryloxaziridine, 2-C₃-C₈-Cycloalkyl-3-aryloxaziridine oder die entsprechenden -3-heteroaryloxaziridine der Formel (II) 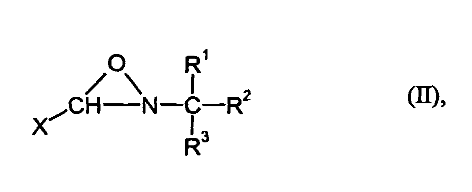 in der die verwendeten Symbole die bei Formel (I) angegebene Bedeutung haben,
herstellt.

3. Verfahren nach Anspruch 2, **dadurch gekennzeichnet, dass** in den Formeln (I) und (II) R¹, R², R³ unabhängig voneinander für Wasserstoff, geradkettiges oder verzweigtes C₁-C₁₀-Alkyl, C₃-C₆-Cycloalkyl, geradkettiges oder verzweigtes C₃-C₆-Alkenyl oder Phenyl oder die gesamte C(R¹)(R²)(R³)-Gruppe für C₃-C₆-Cycloalkyl stehen, wobei die genannten Reste nicht weiter substituiert sind und
X für Phenyl, Naphthyl oder Furyl steht, wobei Phenyl- und Naphthylreste gegebenenfalls durch einen oder zwei gleiche oder verschiedene Reste aus der Gruppe C₁-C₆-Alkyl, Fluor, Chlor, Brom, Hydroxy, C₁-C₆-Alkoxycarbonyl, Nitro, Sulfonyl und Nitril substituiert sein können.

4. Verfahren nach Anspruch 1 bis 3, **dadurch gekennzeichnet, dass** man als Percarbonsäure oder deren Salz m-Chlorperbenzoesäure, Monoperoxyphthalsäure oder deren Alkali- oder Magnesiumsalze einsetzt.

5. Verfahren nach Ansprüchen 1 und 4, **dadurch gekennzeichnet, dass** man als wasserlösliche Basen Alkali- oder Erdalkalioxide, Hydroxide, Carbonate, Bicarbonate, Hydrogenphosphate oder Dihydrogenphosphate einsetzt.

6. Verfahren nach Ansprüchen 1 bis 5, **dadurch gekennzeichnet, dass** man als mit Wasser mischbare Lösungsmittel ein- oder mehrwertige Alkohole mit bis zu 6 C-Atomen einsetzt.

7. Verfahren nach Ansprüchen 2 bis 6, **dadurch gekennzeichnet, dass** man zunächst eine Mischung aus dem Aldimin der Formel (I), in dem mit Wasser mischbaren Lösunsmittel herstellt, die 5 bis 80 Gew.-% des Aldimins der Formel (I) enthält, diese Mischung mit der wässrigen Lösung einer Base umsetzt, die 15 bis 30 Gew.-% Base enthält, zu der so hergestellten Mischung eine 1 bis 20 gew.-%ige Lösung einer aromatischen Percarbonsäure oder deren Salz in einer solchen Dosierrate hinzufügt, das die Reaktionstemperatur 30°C nicht übersteigt, bei 5 bis 30°C nachrührt bis die Reaktion beendet ist und das Reaktionsgemisch mittels Phasentrennung oder Extraktion aufarbeitet.

8. Verfahren nach Ansprüchen 2 bis 7, **dadurch gekennzeichnet, dass** man bezogen auf eingesetztes Aldimin der Formel (I) 0,09 bis 2 Äquivalente Base und 0,9 bis 1,2 Äquivalente aktiven Sauerstoff in Form der aromatischen Percarbonsäure oder deren Salz einsetzt.

## Claims

1. Process for preparing 2-alkyl-3-aryloxaziridines, 2-C₃-C₈-cycloalkyl-3-aryloxaziridines or the corresponding -3-heteroaryloxaziridines, **characterized in that** corresponding N-alkylarylaldimines, N-C₃-C₈-cycloalkylarylaldimines or corresponding -heteroarylaldimines are oxidized using an aromatic percarboxylic acid or a salt thereof in the presence of a watersoluble base at temperatures below 30°C, where the solvent used may be exclusively water and a water-miscible solvent and where all alkyl, aryl and heteroaryl radicals mentioned may optionally be mono- or polysubstituted.

2. Process according to Claim 1, **characterized in that** an N-alkylarylaldimine, N-C₃-C₈-cycloalkylarylaldimine or corresponding -heteroarylaldimine of the formula (I) is used 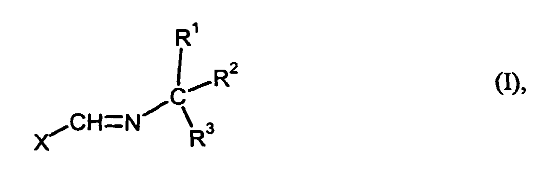 in which
R¹, R² and R³ independently of one another each represent hydrogen, straight-chain or branched C₁-C₂₀-alkyl, C₃-C₈-cycloalkyl, straight-chain or branched C₂-C₁₀-alkenyl or C₆-C₁₀-aryl, or
the entire C(R¹)(R²)(R³) group represents a C₃-C₈-cycloalkyl radical and
X represents C₆-C₁₂-aryl or heteroaryl having 4 or 5 C atoms and 1 or 2 identical or different heteroatoms from the group consisting of N, O and S,
where all alkyl, cycloalkyl, alkenyl, aryl and heteroaryl radicals mentioned may optionally be mono- or polysubstituted,
alkyl radicals by saturated C₃-C₁₂-cycloalkyl, C₆-C₁₀-aryl, C₂-C₈-alkinyl, fluorine, chlorine, bromine, iodine, hydroxyl, C₁-C₆-alkoxy, C₆-C₁₀-aryloxy, carboxyl, C₁-C₆-alkoxycarbonyl, nitro, amido, nitrile, sulphonyl or phosphate and
cycloalkyl, alkenyl, aryl and heteroaryl radicals by C₁-C₆-alkyl, fluorine, chlorine, bromine, hydroxyl, C₁-C₆-alkoxy, carboxyl, C₁-C₆-alkoxycarbonyl, nitro, sulphonyl or nitrile,
and the corresponding 2-alkyl-3-aryloxaziridines, 2-C₃-C₈-cycloalkyl-3-aryloxaziridines or the corresponding -3-heteroaryloxaziridines of the formula (II) 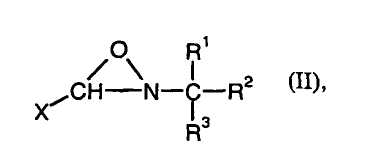 in which the symbols used are as defined under formula (I)
are prepared.

3. Process according to Claim 2, **characterized in that** in the formulae (I) and (II), R¹, R², R³ independently of one another each represent hydrogen, straight-chain or branched C₁-C₁₀-alkyl, C₃-C₆-cycloalkyl, straight-chain or branched C₃-C₆-alkenyl or phenyl or the entire C(R¹)(R²)(R³) group represents C₃-C₆-cycloalkyl, the radicals mentioned not being substituted any further, and,
X represents phenyl, naphthyl or furyl, where the phenyl and naphthyl radicals may optionally be substituted by one or two identical or different radicals from the group consisting of C₁-C₆-alkyl, fluorine, chlorine, bromine, hydroxyl, C₁-C₆-alkoxycarbonyl, nitro, sulphonyl and nitrile.

4. Process according to any of Claims 1 to 3, **characterized in that** the percarboxylic acid or the salt thereof used is m-chloroperbenzoic acid, monoperoxyphthalic acid or their alkali metal or magnesium salts.

5. Process according to any of Claims 1 to 4, **characterized in that** the watersoluble bases used are alkali metal or alkaline earth metal oxides, hydroxides, carbonates, bicarbonates, hydrogenphosphates or dihydrogenphosphates.

6. Process according to any of claims 1 to 5, **characterized in that** the water-miscible solvents used are mono- or polyhydric alcohols having up to 6 C atoms.

7. Process according to any of Claims 2 to 6, **characterized in that** initially a mixture of the aldimine of the formula (I) in the water-miscible solvent is prepared which comprises 5 to 80% by weight of the aldimine of the formula (I), this mixture is reacted with the aqueous solution of a base which comprises 15 to 30% by weight of base, a 1 to 20% by weight strength solution of an aromatic percarboxylic acid or a salt thereof is added to the resulting mixture at such a rate that the reaction temperature does not exceed 30°C, stirring is continued at from 5 to 30°C until the reaction has ended and the reaction mixture is worked up by phase separation or extraction.

8. Process according to any of claims 2 to 7, **characterized in that**, based on the aldimine of the formula (I) used, from 0.09 to 2 equivalents of base and from 0.9 to 1.2 equivalents of active oxygen in the form of the aromatic percarboxylic acid or its salt are used.

## Revendications

1. Procédé pour la préparation de 2-alkyl-3-aryloxaziridines, 2-C₃-C₈-cycloalkyl-3-aryloxaziridines ou des -3-hétéroaryloxaziridines correspondantes, **caractérisé en ce que** l'on oxyde des N-alkylarylaldimines, N-C₃-C₈-cycloalkyl-arylaldimines correspondantes ou -hétéroarylaldimines correspondantes avec un acide percarboxylique aromatique ou un sel de celui-ci, en présence d'une base soluble dans l'eau à des températures inférieures à 30°C, de l'eau et un solvant miscible à l'eau étant exclusivement utilisés comme solvant et tous les restes alkyle, aryle ou hétéroaryle cités pouvant être éventuellement substitués une ou plusieurs fois.

2. Procédé selon la revendication 1, **caractérisé en ce que** l'on utilise une N-alkyl-arylaldimine, N-C₃-C₈-cycloalkyl-arylaldimine ou -hétéroarylaldimine correspondante de la formule (I) 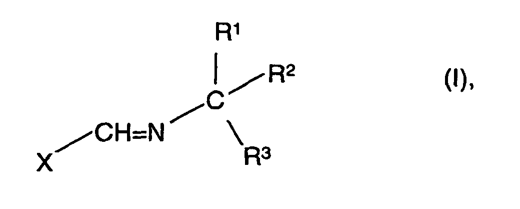 dans laquelle
R¹, R² et R³ représentent indépendamment un atome d'hydrogène, un groupe alkyle en C₁-C₂₀ linéaire ou ramifié, cycloalkyle en C₃-C₈, alcényle en C₂-C₁₀ linéaire ou ramifié ou aryle en C₆-C₁₀, ou le groupe C(R¹)(R²)(R³) entier représente un reste cycloalkyle en C₃-C₈ et
X représente un groupe aryle en C₆-C₁₂ ou hétéroaryle avec de 4 à 5 atomes de C et 1 à 2 hétéroatomes identiques ou différents de la série N, O et S,
tous les restes alkyle, cycloalkyle, alcényle, aryle et hétéroaryle cités pouvant être éventuellement substitués une ou plusieurs fois,
les restes alkyle par un groupe cycloalkyle en C₃-C₁₂, aryle en C₆-C₁₀, alcynyle en C₂-C₈ saturé, un atome de fluor, de chlore, de brome, d'iode, un groupe hydroxy, alcoxy en C₁-C₆, aryloxy en C₆-C₁₀, carboxy, (alcoxy en C₁-C₆)carbonyle, nitro, amido, nitrile, sulfonyle ou phosphate et
les restes cycloalkyle, alcényle, aryle et hétéroaryle par un groupe alkyle en C₁-C₆, un atome de fluor, de chlore, de brome, un groupe hydroxy, alcoxy en C₁-C₆, carboxy, (alcoxy en C₁-C₆)carbonyle, nitro, sulfonyle ou nitrile
et on prépare les 2-alkyl-3-aryloxaziridines, 2-C₃-C₈-cycloalkyl-3-aryloxaziridines correspondantes ou -3-hétéroaryloxaziridines correspondantes de la formule (II) 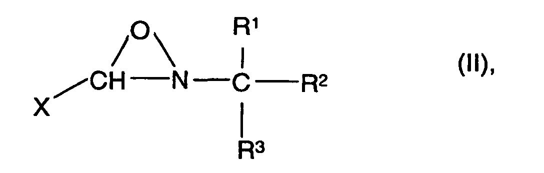 dans laquelle les symboles utilisés ont la signification indiquée pour la formule (I).

3. Procédé selon la revendication 2, **caractérisé en ce que** dans les formules (I) et (II), R¹, R², R³ représentent indépendamment un atome d'hydrogène, un groupe alkyle en C₁-C₁₀ linéaire ou ramifié, cycloalkyle en C₃-C₆, alcényle en C₃-C₆ linéaire ou ramifié ou phényle et le groupe C(R¹)(R²)(R³) entier représente un groupe cycloalkyle en C₃-C₆, les restes cités n'étant pas plus substitués et
X représente un groupe phényle, naphtyle ou furyle, les restes phényle et naphtyle pouvant être éventuellement substitués par un ou deux restes identiques ou différents choisis parmi un groupe alkyle en C₁-C₆, un atome de fluor, de chlore, de brome, un groupe hydroxy, alcoxycarbonyle en C₁-C₆, nitro, sulfonyle et nitrile.

4. Procédé selon les revendications 1 à 3, **caractérisé en ce que** l'on utilise comme acide percarboxylique ou sel de celui-ci, l'acide m-chloroperbenzoïque, l'acide monoperoxyphtalique ou des sels d'alcalis ou de magnésium de ceux-ci.

5. Procédé selon les revendications 1 et 4, **caractérisé en ce que** l'on utilise comme bases solubles dans l'eau des oxydes, hydroxydes, carbonates, bicarbonates, hydrogénophosphates ou dihydrogénophosphates alcalins ou alcalino-terreux.

6. Procédé selon les revendications 1 à 5, **caractérisé en ce que** l'on utilise comme solvants miscibles à l'eau des alcools mono ou polyvalents avec jusqu'à 6 atomes de C.

7. Procédé selon les revendications 2 à 6, **caractérisé en ce que** l'on prépare dans un premier temps un mélange de l'aldimine de la formule (I) dans le solvant miscible à l'eau, lequel contient de 5 à 80 % en poids de l'aldimine de la formule (I), on fait réagir ce mélange avec la solution aqueuse d'une base qui contient de 15 à 30 % en poids de base, on ajoute au mélange ainsi préparé une solution à de 1 à 20 % en poids d'un acide percarboxylique aromatique ou d'un sel de celui-ci, à une vitesse de dosage telle que la température de réaction ne dépasse pas 30°C, on continue à agiter à de 5 à 30°C jusqu'à ce que la réaction soit achevée et on traite le mélange réactionnel au moyen d'une séparation de phases ou d'une extraction.

8. Procédé selon les revendications 2 à 7, **caractérisé en ce que** l'on utilise, rapportés à l'aldimine utilisée de la formule (I), de 0,09 à 2 équivalents de base et de 0,9 à 1,2 équivalent d'oxygène actif dans la forme de l'acide percarboxylique aromatique ou d'un sel de celui-ci.
